(19) Europäisches Patentamt / European Patent Office / Office européen des brevets

(11) EP 2 366 664 A1

(12) EUROPEAN PATENT APPLICATION

(43) Date of publication:
21.09.2011 Bulletin 2011/38

(21) Application number: 10002419.9

(22) Date of filing: 09.03.2010

(51) Int Cl.:
$C01F\ 7/00$ (2006.01)
$C01G\ 55/00$ (2006.01)
$B01J\ 23/62$ (2006.01)
$C07C\ 5/05$ (2006.01)
$C22C\ 5/04$ (2006.01)
$B01J\ 23/44$ (2006.01)
$B01J\ 23/60$ (2006.01)
$B01J\ 37/16$ (2006.01)
$C07C\ 5/09$ (2006.01)

(84) Designated Contracting States:
AT BE BG CH CY CZ DE DK EE ES FI FR GB GR
HR HU IE IS IT LI LT LU LV MC MK MT NL NO PL
PT RO SE SI SK SM TR
Designated Extension States:
AL BA ME RS

(71) Applicant: Max-Planck-Gesellschaft zur
Förderung
der Wissenschaften e.V.
80539 München (DE)

(72) Inventors:
• Behrens, Malte
14163 Berlin (DE)

• Ota, Antje
16767 Leegebruch (DE)
• Schlögl, Robert, Prof. Dr.
10779 Berlin (DE)
• Armbrüster, Marc Dr.
01187 Dresden (DE)
• Grin, Juri Dr.
01277 Dresden (DE)

(74) Representative: HOFFMANN EITLE
Patent- und Rechtsanwälte
Arabellastraße 4
81925 München (DE)

(54) **Palladium-modified hydrotalcites and their use as catalyst precursors**

(57) The present invention relates to hydrotalcite-like compounds, wherein $Pd^{2+}$ occupies at least part of the octrahedral sites in the brucite-like layers. According to another aspect, the invention is concerned with methods of converting these hydrotalcite-like compounds into materials comprising particles, in particular nanoparticles, of an ordered intermetallic compound of palladium and at least one constituent metal of the palladium-modified hydrotalcites. Moreover, the invention pertains to the material obtainable by the conversion method, the use of the material as a catalyst, and a process for the selective hydrogenation of alkyne(s) to the corresponding alkene (s) using the material as a hydrogenation catalyst.

Fig. 1

**Description**

FIELD OF THE INVENTION

The present invention relates to palladium-modified hydrotalcites, and methods for the preparation thereof. Furthermore, the present invention is concerned with methods of converting the palladium-modified hydrotalcites into a material comprising particles of an ordered intermetallic compound of palladium and at least one constituent metal of the palladium-modified hydrotalcites, as well as the material obtainable by the method, the use of the material as a catalyst, and a process for the selective hydrogenation of alkyne(s) to the corresponding alkene(s) using the material as a hydrogenation catalyst.

BACKGROUND ART

The heterogeneously catalyzed semi-hydrogenation of acetylene (ethyne) is an important industrial purification step of the ethylene (ethene) feed for the production of polyethylene. The selectivity of the catalyst is crucial. First of all, the acetylene content in the ethylene feed has to be reduced from approximately 1% to low ppm levels. This is because remaining acetylene will poison the polymerization catalyst in the subsequent polymerization step to give polyethylene. Furthermore, the loss of valuable ethylene by hydrogenation to ethane has to be avoided.

Typical hydrogenation catalysts contain palladium dispersed on metal oxides. While palladium metal exhibits high activity, e.g. in the hydrogenation of acetylene, it possesses only limited selectivity and stability because of the formation of ethane through complete hydrogenation, and the formation of $C_4$ and higher hydrocarbons by oligomerization reactions.

Various attempts have been made to enhance the selectivity of palladium catalysts in the selective hydrogenation of alkynes, in particular acetylene.

One approach was the concept of active site isolation. For instance, the isolation of the active palladium hydrogenation sites was realized by alloying. Pd20Ag80 is such an alloy.

A different and just recently introduced approach is the use of structurally well-ordered intermetallic compounds. Such catalysts are described in WO 2007/104569 and the corresponding EP-A-1 834 939. They comprise at least one hydrogenation-active type of metal and at least one type of metal not capable of activating hydrogen. PdGa and $Pd_3Ga_7$ proved to be highly selective catalysts in the selective hydrogenation of acetylene to ethylene (see also J. Osswald, J. of Catal. 258 (2008) 210 and J. Osswald et al., J. of Catal. 258 (2008) 219). In the catalytic tests, unsupported intermetallic palladium-gallium compounds obtained by melting together the necessary amounts of palladium and gallium were used. For samples obtained by melting together the constituent metals with further treatment in a swing mill or subsequent chemical etching using aqueous ammonia solution the catalytic activity was improved. Nevertheless, there was still room for improvement.

Furthermore, the activity of the ordered intermetallic compounds, e.g. binary ordered palladium-gallium intermetallic compounds, could be increased while retaining the high selectivity level by mixing the ordered intermetallic compounds with inert materials, such as alumina and silica. See WO 2009/037301 and the corresponding priority application EP-A-2 039 669. While the activity could be improved this way, there was still room for improvement.

With the aim of enhancing the catalytic activity of the ordered intermetallic palladium-gallium compounds, a method of preparing these compounds was proposed in EP-A-2 060 323 and the corresponding WO 2009/062848 which involved the co-reduction of a palladium compound and a gallium compound with a reducing agent. For instance, the co-reduction of $Pd(acac)_2$ and $GaCl_3$ with Superhydride® (1.0 M lithium triethyl borohydride in THF) in tetrahydrofurane (THF) under inert atmosphere yielded, depending on the initial palladium-gallium ratio, PdGa or $Pd_2Ga$ nanoparticles, which were shown to have increased activities with excellent selectivities as compared to bulk materials being maintained. Unfortunately, the above nanoparticle synthesis is less attractive to industry due to the high costs of the starting compounds as well as the need of an inert atmosphere during synthesis.

It is to be noted that conventional methods to synthesize multi-metal catalysts, e.g. by wet-impregnation, while being inexpensive, will not result in single-phase supported palladium-gallium intermetallic compounds. Such a conventional approach is pursued by T. Komatsu et al. in Appl. Catal. A 251 (2003) 315. Following the catalyst preparation techniques of that article, an uncontrolled mixture of species is generally obtained, which comprises pure elemental palladium so that the selectivity of these catalysts is not satisfactory.

In the new approach of the present invention, palladium-modified hydrotalcites are used as precursors for supported palladium-gallium intermetallic compound catalysts.

F. Cavani et al. provide in Catal. Today 11 (1991) 173 a comprehensive review of hydrotalcites and hydrotalcite-like compounds. An overview of the physicochemical characterization of hydrotalcite-like compounds is given, and catalytic applications of hydrotalcite-like compounds are summarized. In the review article, hydrotalcite-like compounds are defined as having the following formula: $[M(II)_{1-x}M(III)_x(OH)_2]^{x+}(A^{n-}_{x/n}) \cdot mH_2O$, wherein A represents an interlamellar

anion; $0.1 \leq x \leq 0.5$, especially $0.2 \leq x \leq 0.33$; and n is the charge of the anion A. M(II) and M(III) represent divalent and trivalent metal ions. Only M(II) and M(III) ions having a certain maximum size will fit into the structure. Specifically, the following cations M(II) are stated as being capable of forming hydrotalcite-like compounds: $Mg^{2+}$, $Cu^{2+}$, $Ni^{2+}$, $Co^{2+}$, $Zn^{2+}$, $Fe^{2+}$ and $Mn^{2+}$. M(II) cations having an ionic radius (for a coordination number of 6) larger than that of $Mn^{2+}$ (83 pm) are considered as too big to form hydrotalcite-like structures. For instance, $Ca^{2+}$ having an ionic radius of 100 pm for the coordination number 6 is too big for it to be incorporated in hydrotalcite-like structures. As far as the present Applicant is aware, prior to the present invention, no one has ever accomplished to accommodate $Pd^{2+}$ having an ionic radius of 86 pm for a coordination number of 6 as M(II) cations into hydrotalcite-like structures. Pursuant to F. Cavani et al., in the case of M(III), the following variations are possible: $Al^{3+}$, $Ga^{3+}$, $Ni^{3+}$, $Co^{3+}$, $Fe^{3+}$, $Mn^{3+}$ and $Cr^{3+}$. The calcination of hydrotalcite-like compounds will allow the formation of homogeneous mixtures of oxides with very small crystal size, which by reduction form small and thermally stable metal crystallites.

Gallium-containing hydrotalcite-like materials are also described by E. Lopéz-Salinas et al. in J. Phys. Chem. B. 101 (1997) 5112 and in J. Porous Mater. 3 (1996), 169.

Hydrotalcite-like structures as precursors of hydrogenation catalysts are dealt with in DE-A-2 024 282.

A. Monzón, in Appl. Catalysis A 185 (1999) 53 describe the use of hydrotalcites or hydrotalcite-like compounds as catalytic precursors of multi-metallic mixed oxides, as well as their application in the hydrogenation of acetylene. The metal cations involved in the study described in the paper are Ni, Zn, Al, Cr and Fe.

In summary, hydrotalcite-like compounds have occasionally been used as precursors for the preparation of well-dispersed metal oxide catalysts and, after reduction treatment, metal catalysts. However, prior to the present invention, hydrotalcite-like compounds have, to the best of the Applicant's knowledge, not yet been used as precursors for the preparation of catalysts comprising highly dispersed ordered intermetallic compounds. In particular, it could not be expected that the novel palladium-modified hydrotalcites of the present invention can serve as precursors for catalysts showing excellent activity and selectivity in selective hydrogenation reactions.

In view of the above, it is an object of the invention to provide novel catalysts being both, highly active and highly selective in the hydrogenation of alkyne(s) to the corresponding alkene(s), in particular the semi-hydrogenation of acetylene to ethylene. It is a further aspect of the present invention to provide such catalysts, which are inexpensive to prepare and as such useful in industry.

SUMMARY OF THE INVENTION

The present invention is based on the finding that palladium can be incorporated in the structure of hydrotalcite-like materials. The present invention is, according to a first aspect, concerned with a hydrotalcite-like compound, wherein $Pd^{2+}$ occupies at least part of the octahedral sites in the brucite-like layers. In the present specification, these hydrotalcite-like compounds will occasionally be referred to as "palladium-modified hydrotalcites" or simply as "Pd-hydrotalcites".

The Pd-hydrotalcites according to the present invention open up a new route of preparing supported particulate ordered intermetallic compounds, especially in the form of supported nanoparticles. Such materials are easily accessible by reduction of the Pd-hydrotalcites, preferably with a hydrogen-containing gas, at temperatures as recited in the appending Claim 8. The material comprising particles of an ordered intermetallic palladium compound obtainable by the method of Claim 8 will be referred to herein as "Pd-hydrotalcite derived material". The Pd-hydrotalcite derived material is a second aspect of the present invention.

While easily accessible from the Pd-hydrotalcites (serving as precursor materials), the Pd-hydrotalcite derived materials proved to be highly selective and active catalysts, e.g. in the selective hydrogenation of alkyne(s) to the corresponding alkene(s), in particular in the selective semi-hydrogenation of acetylene to ethylene. Such a process, which is claimed in Claim 13, comprises the reaction of the alkyne(s), preferably acetylene, with hydrogen in the presence of a Pd-hydrotalcite derived material in accordance with the present invention. In more general terms, the present invention is also concerned with the use of the Pd-hydrotalcite derived material according to the present invention as a catalyst.

Preferred embodiments of the present invention are subject of the dependent claims.

BRIEF DESCRIPTION OF THE DRAWINGS

Fig. 1 provides a schematic general representation of a hydrotalcite-like structure with interlayer carbonate anions (interlayer water molecules are not shown for clarity).

Fig. 2 provides a schematic representation of brucite-like layers in hydrotalcite-like structures, viewed perpendicular to the layers (Fig. 2a) and parallel to the layers (Fig. 2b).

Fig. 3 shows several X-ray powder diffraction (XRD) patters: the theoretical pattern of MgGa hydrotalcite (A), the experimental pattern of a palladium-free MgGa-hydrotalcite (for comparison) (B), and the experimental pattern of a PdMgGa-hydrotalcite in accordance with the present invention (C).

Fig. 4 is showing the conversion and selectivity of the Pd-hydrotalcite derived material obtained in Example 2 in the selective hydrogenation of acetylene in admixture with an excess of ethylene at 200°C to give ethylene.

Fig. 5 is the X-ray powder diffraction pattern of the Pd-hydrotalcite derived material obtained in Example 1. For comparison, the positions of the main reflections of $Pd_2Ga$ (Powder Diffraction File PDF [65-1511]) are shown as filled columns, and those of MgO (PDF [1-1235]) as unfilled, i.e. white columns.

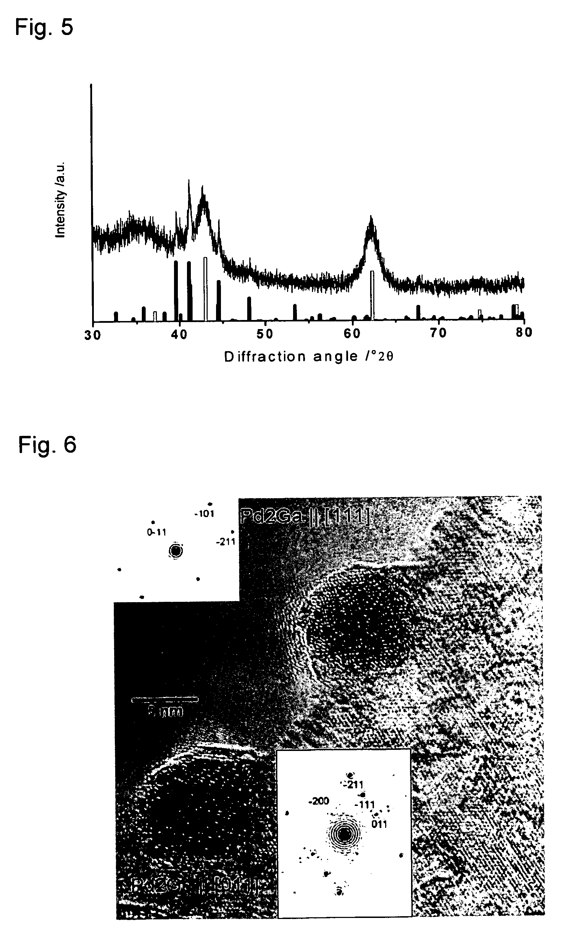

Fig. 6 is a HRTEM (high resolution transmission electron microscopy) photograph of a Pd-hydrotalcite derived material in accordance with the present invention after reduction of a Pd-hydrotalcite precursor material in a flow of 5% hydrogen in argon at 550 °C for 4 h. For the synthesis of the Pd-hydrotalcite precursor material in accordance with the general preparation method described in the Examples Section below, 1 mol% palladium in relation to the overall molar amount of palladium, magnesium and gallium was used in the initial salt mixture. The upper left insert of Fig. 6 shows the electron diffraction pattern measured with respect to the [111] plane of $Pd_2Ga$, and the lower insert the electron diffraction pattern with respect to the [011] plane of $Pd_2Ga$.

## DETAILED DESCRIPTION OF THE INVENTION

In the present invention, the term "hydrotalcite-like compound" is used as a generic term encompassing all compounds having the same basic structure as hydrotalcite as such. Hydrotalcite has the formula $Mg_4Al_2(OH)_{12}CO_3 \cdot 4H_2O$. Hydrotalcite-like compounds are occasionally also referred to as "layered double hydroxides", abbreviated "LDH", in the literature.

The crystal structure of hydrotalcite, and consequently also hydrotalcite-like compounds is derived from brucite, i.e. $Mg(OH)_2$. The structure of brucite is built up as follows. $Mg^{2+}$ is octahedrally coordinated by hydroxyl (OH) groups. That means, $Mg^{2+}$ is located in the centre of an octahedron, the six corners of which are occupied by hydroxyl groups. In brucite, the octahedra share edges to form layers. These layers are stacked on top of each other and are held together by hydrogen bonding.

When $Mg^{2+}$ ions are substituted by a trivalent cation having not too different a radius, a positive charge is generated in the layers of brucite. For instance, in the parent compound hydrotalcite a net positive charge originates from partial replacement of $Mg^{2+}$ by $Al^{3+}$. Such layers having a net positive charge that are derived from brucite layers by replacement of $Mg^{2+}$ by a trivalent metal cation of appropriate size, i.e. not too different from $Mg^{2+}$ are referred to as "brucite-like layers" in this specification. In the alternative, they could also be called "brucitic layers". Examples of suitable trivalent metal cations in brucite-like layers are $Al^{3+}$, $Ga^{3+}$, $Ni^{3+}$, $Co^{3+}$, $Fe^{3+}$, $Mn^{3+}$ and $Cr^{3+}$.

When starting from the parent compound hydrotalcite of the formula $Mg_4Al_2(OH)_{12}CO_3 \cdot 4H_2O$, $Al^{3+}$ can be partially or completely replaced by trivalent metal cations of similar size such as $Ga^{3+}$, $Ni^{3+}$, $Co^{3+}$, $Fe^{3+}$, $Mn^{3+}$ and $Cr^{3+}$ and independently $Mg^{2+}$ can be replaced by divalent cations of similar size, such as $Ni^{2+}$, $Co^{2+}$, $Zn^{2+}$, $Fe^{2+}$, $Cu^{2+}$ and $Mn^{2+}$.

In hydrotalcite and hydrotalcite-like compounds, the net positive charge in the brucite-like layers is compensated for by anions, which lie between two brucite-like layers. Such anions will occasionally be denoted "interlayer anions" herein. In the case of the parent hydrotalcite-like compound, namely, hydrotalcite, the interlayer anion is carbonate. In the space between two brucite-like layers, also water finds a place. This is occasionally denoted "interlayer water" in this specification.

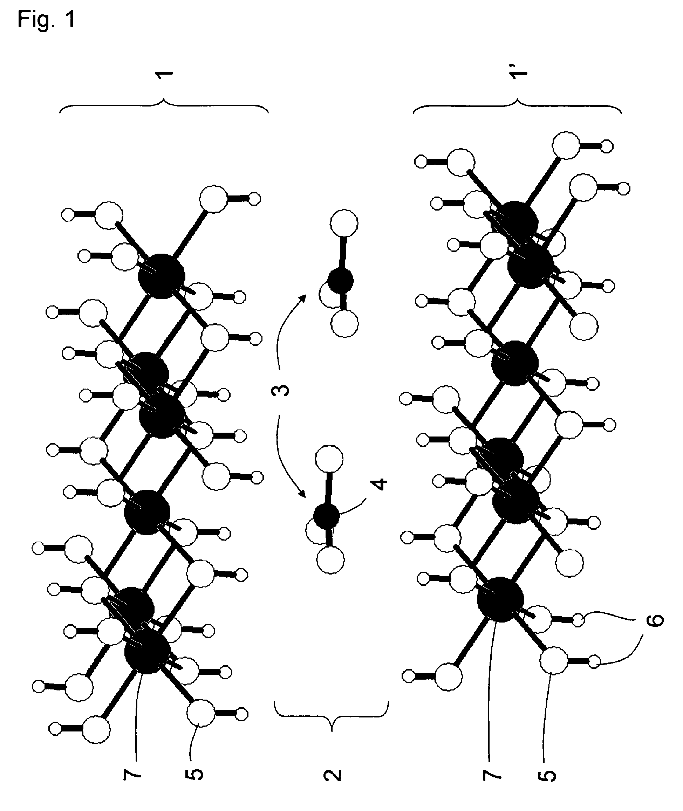

Fig. 1 provides a general representation of the crystal structure of hydrotalcite-like compounds. In the figure, two brucite-like layers 1, 1' are shown. In-between them, the interlayer space or region 2 is formed. Within the interlayer region there are interlayer anions 3. Concretely, carbonate is the interlayer anion in Fig. 1, the black circles 4 denoting carbon atoms, and the open circles oxygen atoms 5. For clarity, interlayer water molecules located in the interlayer space 2 are omitted in Fig. 1. The small white circles represent hydrogen atoms 6, and the large grey circles 7 divalent or trivalent metal cations.

As can be seen from Fig. 1, the divalent and trivalent metal cations 7 occupy the octahedral sites in the brucite-like layers 1. As meant herein, "octahedral sites" in the brucite-like layers refers to the position in the centre of the octahedra formed by six hydroxyl groups in the edge-sharing octahedra of the brucite-like layers.

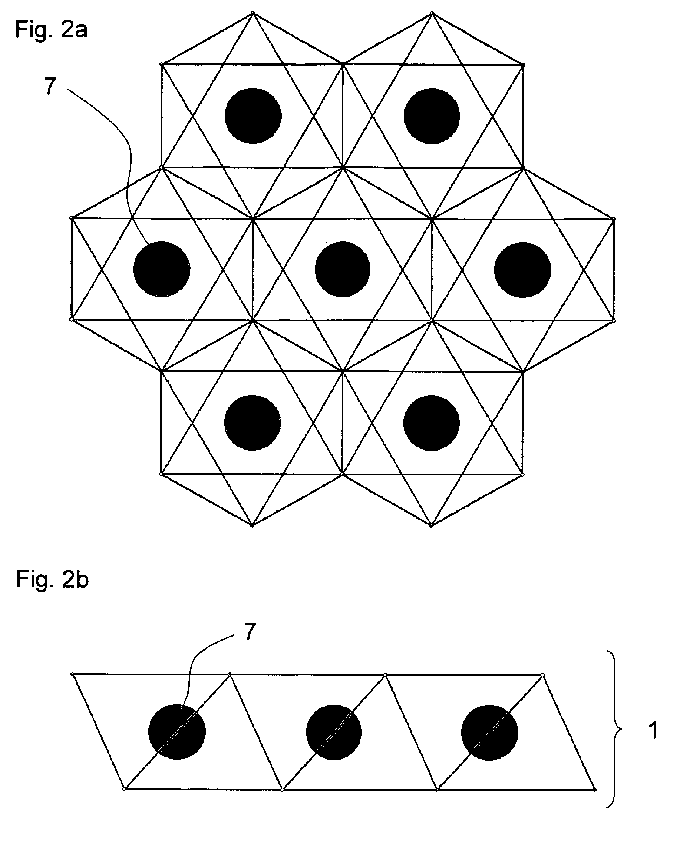

The positioning of the divalent or trivalent metal cations 7 at the octahedral sites in the brucite-like layers is further illustrated in Fig. 2a/b. Fig. 2a provides a top view from above a brucite-like layer, and Fig. 2b a side view. For the sake of clarity, the hydroxyl groups are omitted. They are located at the corners of the octahedra shown in Fig. 2a/b.

In the Pd-hydrotalcites of the invention, at least part of the octahedral sites in the brucite-like layers is occupied by $Pd^{2+}$ ions. Accordingly, at least part of the metal cations 7 in Fig. 1 is $Pd^{2+}$. According to a preferred embodiment, 0.005 to 5%, preferably 0.01 to 1%, more preferably 0.05 to 1% of the octahedral sites in the brucite-like layers is occupied by $Pd^{2+}$.

Such ratios of palladium in the octahedral sites of the brucite-like layers translate into a Pd content of 0.0015 to 12.7% by weight in the Pd-hydrotalcite of the present invention.

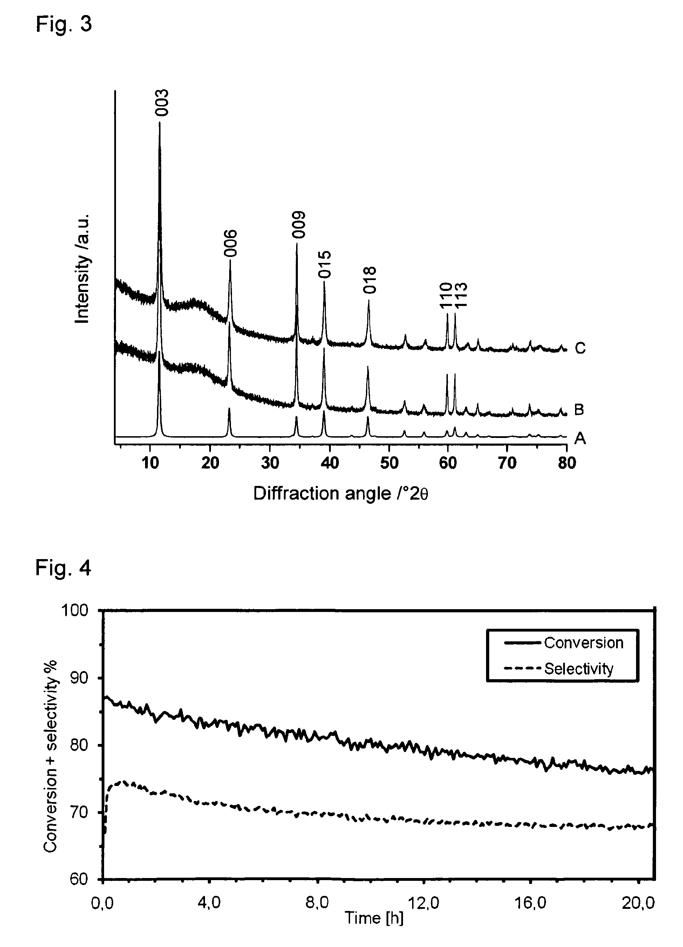

To verify that the Pd-hydrotalcites of the invention have a hydrotalcite-like structure as explained above by reference to Figs. 1 and 2, X-ray powder diffraction analysis (XRD) turned out to be useful. Typical XRD patterns are shown in Fig. 3, in which the intensity is given in arbitrary units (a.u.). Pattern (A) is the theoretical pattern of a MgGa-hydrotalcite; pattern (B) was obtained with a palladium-free MgGa-hydrotalcite serving for comparison; and (C) is the XRD pattern of a PdMgGa-hydrotalcite in accordance with the present invention. In Fig. 3, the vertical numbers (such as "003") indicate the Miller indices of the main reflections.

In addition, the thermal properties of the Pd-hydrotalcites of the invention were shown to be typical for hydrotalcite-like compounds. This was verified by thermogravimetric-mass spectroscopic analysis (TG-MS). Specifically, the liberation of the interlayer water is observed in a well defined dehydration step, followed by dehydroxylation at temperatures of below 450 °C in several steps. When carbonate is the interlayer anion, this is decomposed in a broad temperature range up to about 600 °C.

According to a preferred embodiment, the Pd-hydrotalcite of the invention is represented by the following formula (I):

$$[(Pd^{2+}, M2)_{1-x} M3_x (OH)_2]^{x+} (A^{n-}_{x/n}) \cdot m\, H_2O \qquad (I),$$

wherein:

M2 is at least one divalent metal cation selected from the group consisting of $Mg^{2+}$, $Ni^{2+}$, $Co^{2+}$, $Zn^{2+}$, $Fe^{2+}$, $Cu^{2+}$ and $Mn^{2+}$;

M3 is at least one trivalent metal cation selected from $Al^{3+}$, $Ga^{3+}$, $Ni^{3+}$, $Co^{3+}$, $Fe^{3+}$, $Mn^{3+}$ und $Cr^{3+}$;

A is an n-valent anion, preferably carbonate;

x is 0.1 - 0.5, preferably $0.2 \leq x \leq 0.33$; and

m is 0.1-1.0.

In the above formula (I), M2 and M3 can independently be mixtures of divalent and trivalent metal cations, respectively.

For the sake of conciseness, specific Pd-hydrotalcites represented by the above formula (I) will be named below PdM2M3-hydrotalcites. To give an example, a Pd-hydrotalcite according to the invention, wherein M2 is $Mg^{2+}$ and M3 is $Ga^{3+}$ can be denoted "PdMgGa-hydrotalcite".

While not specifically limited, with an eye on the palladium content in the Pd-hydrotalcite derived material obtainable from the Pd-hydrotalcite of the invention through reduction, the ratio of $Pd^{2+}$ to M2 ($Pd^{2+}/M2$) in the Pd-hydrotalcite may be in the range of 0.0001 to 0.1.

There are no specific restrictions as to the n-valent anion A, and inorganic anions can for instance be used. Examples are $F^-$, $Cl^-$, $Br^-$, $I^-$, $(ClO_4)^-$, $(NO_3)-$, $(ClO_3)^-$, $(IO_3)-$, $OH^-$, $(CO_3)^{2-}$, $(SO_4)^{2-}$, $(S_2O_3)^{2-}$, $(WO_4)^{2-}$, $(CrO_4)^{2-}$, $[Fe(CN)_6]^{3-}$, $[Fe(CN)_6]^{4-}$ and $[SiO(OH)_3]^-$. Anions of organic acids, such as adipic, oxalic, succinic, malonic, sebacic and 1,12-dodecanedicarboxylic acid can also be used. Of course, a mixture of anions can also be used as the interlayer anion(s), represented in the above formula (I) by A. In view of the ease of manufacturing, carbonate ($CO_3^{2-}$) (with n being 2) is the most preferred interlayer anion in the Pd-hydrotalcites of the invention.

The palladium-modified hydrotalcite-like compounds of the invention can be prepared by co-precipitation of the constituent metal cations and anions. In order to achieve a co-precipitation of the cations, conditions of supersaturation have to be fulfilled. In the preparation method of the present invention, supersaturation conditions can be reached by physical methods, such as evaporation, or chemical methods, such as variation of pH. For the preparation of the Pd-hydrotalcites according to the present invention, the pH variation to reach a supersaturated state in order to co-precipitate the Pd hydrotalcite proved to be advantageous. In that method, the precipitation of the metal cations is carried out at a pH higher than or equal to the one at which the more soluble of the hydroxides of the metals forming the hydrotalcite-like structure precipitates. The pH of precipitation of hydroxides such as M2 and M3 hydroxides (for the meaning of M2 and M3 see formula (I)) are known in the art. The pH useful to precipitate the Pd hydrotalcites of the invention may for instance be in the range of 8 to 10. In the art of preparing hydrotalcite-like compounds, three methods of precipitation have been used:

1) Titration with NaOH and/or $NaHCO_3$, often referred to as sequential precipitation or increasing pH method;

2) Precipitation at low supersaturation at constant pH; and

3) Precipitation at high supersaturation at constant pH.

For more details of the above general methods of preparing hydrotalcite-like compounds, reference can be made to the review article by F. Cavani in Catalysis Today, 11 (1991) 173-301.

While the general methods of preparing hydrotalcite-like compounds as summarized above are also applicable to the Pd-hydrotalcites according to the present invention, the above method (2) is preferred. That method will therefore be further described. In that method, water-soluble salts of the constituent metal cations of the Pd-hydrotalcite are dissolved in an aqueous solvent to prepare an aqueous solution. Nitrates of the metal cations Pd(II), M2 and M3 are used with preference in that the nitrate anion will not contaminate the Pd-hydrotalcite product. To the thus-obtained aqueous solution, the (interlayer) anion(s) are added to precipitate the Pd hydrotalcite. The anions are preferably added in the form of an aqueous solution. According to a preferred embodiment, the pH of the aqueous solution of the constituent cations of the Pd-hydrotalcite is controlled during the addition of the solution of the interlayer anion(s). Thereby, the pH is preferably kept in a range of 8 to 10.

In a particularly preferred embodiment, the pH is controlled within that range by the slow addition in a single container of two diluted streams, the first stream containing the constituent cations of the Pd-hydrotalcite, such as Pd (II), M2 and M3 cations, and the second stream containing the anion, e.g. for carbonate interlayer anions the base (KOH, NaOH, NaHCO$_3$ and/or Na$_2$CO$_3$).

With the purpose of obtaining pure, single-phase Pd-hydrotalcites according to the invention, it is preferred to choose in the preparation method a ratio of cations and anions in terms of the final Pd-hydrotalcite, as follows:

$$0.2 \leq M3/[Pd^{2+}+M2+M3] \leq 0.4,$$

and

$$1/n \leq A^{n-}/M3.$$

Thereby the meanings of M2, M3, A and n are as defined in connection with formula (I) above.

The temperature during the precipitation of the Pd-hydrotalcites in the preparation method of the invention is not specifically limited, and may for instance be in the range of 20 to 90 °C, preferably 50 to 70 °C.

The precipitated Pd-hydrotalcites may be subjected to ageing prior to separation from the solution, i.e. mother liquor. For instance, the aging in the mother liquor can be carried out under the conditions of precipitation, in particular the same temperature. The separation from the solution can be effected by usual methods, such as filtration.

Subsequently, the Pd-hydrotalcite can be dried. Typical drying temperatures are in the range of 60 to 120 °C, preferably 80 to 100 °C. In the next step, the Pd-hydrotalcites are optionally calcined, for instance at 400°C in air for 4 hours. Typical calcination temperatures are in the range of 150 to 800 °C, preferably 300 to 500 °C.

As the present inventors found, the Pd-hydrotalcites according to the present invention can be converted by simple reduction into a Pd-hydrotalcite derived material, which comprises finely distributed (nano)particles of an ordered intermetallic palladium compound and therefore has remarkable catalytic properties, e.g. in hydrogenation, in particular selective hydrogenation reactions. Accordingly, in the present invention, the Pd-hydrotalcites can be referred to as intermediates or precursors for the preparation of the Pd-hydrotalcite derived material.

The reduction is preferably carried out with a hydrogen-containing gas of a hydrogen concentration typically between 1 and 100 % and at a pressure between ambient and 100 bar. The reduction temperatures may be in the range of 100 to 1000 °C. Preferably, the reduction temperature is 300 to 900 °C, more preferably 350 to 850 °C, still more preferably 500 to 800 °C and most preferably 550 to 700 °C.

Dependent on the particular Pd-hydrotalcite to be converted, suitable reduction temperatures within these ranges can be selected.

Moreover, within the reduction temperature range suitable to a particular Pd-hydrotalcite, temperatures as low as possible are preferred because this will lead to less sintering and consequently smaller sizes of the particles of the ordered intermetallic compound in the resultant Pd-hydrotalcite derived material, with concomitant higher catalytic activity.

For instance, for the conversion of PdZnAl-hydrotalcite in accordance with the invention, reduction temperatures as low as 400 °C proved sufficient. In the case of PdMgGa-hydrotalcites according to the invention, reduction temperatures above 700 °C, e.g. in the most preferred range of 750 to 850 °C will yield upon reduction Pd-hydrotalcite derived material being particularly active and selective in selective hydrogenations.

The Pd-hydrotalcite derived materials of the invention comprise particles of an ordered intermetallic compound of palladium and the further constituent metal cations, such as M2 and/or M3 (wherein M2 and M3 have the meaning as defined for formula (I) above).

As used herein, the term "ordered intermetallic compound" refers to a compound consisting of two or more metals such as palladium and gallium having an ordered crystal structure. In the ordered crystal structure, substantially all unit cells have the same arrangement of metal atoms. As such, the ordered intermetallic compounds are to be distinguished from metal alloys and metal solid solutions. Alloys and solid solutions do not have an ordered atomic structure, as described above. Rather, metal atoms are arranged randomly at the atomic positions in the unit cells of alloys and solid solutions.

It will be appreciated that defects which usually cannot be completely avoided in a real crystal may be present in the intermetallic ordered compound. Such defects can cause a small number of unit cells in the ordered intermetallic compound to have an arrangement of metal atoms different from the majority of the unit cells. Defect types include for example vacancies, interstitials, atom substitutions and anti-site defects.

The formulae used in the present specification refer to the ideal crystal structure. As will be appreciated from the above, the stoichiometric ratio of the metals forming the ordered intermetallic compound as indicated in the formula may vary up and down. To give an example, if the ordered intermetallic compound is represented by the general formula $Pd_xGa_y$, then x and y may independently be an integer of 1 or more. In the present specification, PdGa (i.e. x = y = 1) and $Pd_2Ga$ represent intermetallic Pd/Ga compounds having a certain stoichiometric ratio of the constituent metals palladium and gallium. Taking account of the above homogeneity ranges the values of x and y may be slightly greater or slightly less than the integers indicated in the formula. For instance, the values of x and y may vary by $\pm \varepsilon$, with $\varepsilon$ being in the range of 0.001 to 0.01. The range of the numerical values for the respective ordered intermetallic compound can be taken from the phase diagram of the compound. It corresponds to the respective single-phase region of the intermetallic compound.

For instance starting from a PdMgGa-hydrotalcite, particles of an ordered intermetallic palladium-gallium compound will form. Specifically, depending on the ratio of Pd and M3 = $Ga^{3+}$ in the PdMgGa-hydrotalcite precursor, PdGa or $Pd_2Ga$ will form during the reduction. These particles were shown to be highly dispersed. In fact, it was shown by transmission electron microscopy (TEM) and high resolution transmission electron microscopy (HRTEM) that nanoparticles are formed, which are finely distributed on the carrier, and that these nanoparticles consist of the respective ordered intermetallic compound, such as $Pd_2Ga$. A typical HRTEM photograph of Pd-hydrotalcite derived materials of the invention is shown in Fig. 6. Two nanoparticles can be seen. By measuring the electron diffraction patterns with respect to the [111] plane (cf. upper left insert) and with respect to the [011] plane (cf. bottom insert), it could be verified that the observed nanoparticles are indeed $Pd_2Ga$ nanoparticles.

Apart from HRTEM, the formation of ordered intermetallic compounds, such as $Pd_2Ga$, could be confirmed by way of X-ray powder diffraction (XRD) measurements. Fig. 5 is a typical example. It is evident from a comparison of the measured diffraction pattern with the PDF (Powder Diffraction File) of $Pd_2Ga$ (filled columns) that $Pd_2Ga$ is indeed formed. In addition, the MgO present in the matrix can be seen from the XRD pattern of Fig. 5.

As meant herein, nanoparticles have an average diameter in the nanometer range, i.e. from 1 nm to below 1000 nm. Preferably, the nanoparticles have an average diameter of 1 to 100 nm.

According to a preferred embodiment, the particles of the ordered intermetallic palladium compound in the Pd-hydrotalcite derived material of the invention are single phase particles. That means they consist only of a single specific ordered intermetallic palladium compound.

It is assumed that in the case of a PdMgGa-hydrotalcite precursor, palladium nanoparticles will first form upon decomposition of the Pd-hydrotalcite during reduction. It is speculated that these nanoparticles are presumably capable of adsorbing hydrogen (spillover hydrogen), which will reduce the $Ga^{3+}$ located in the neighbourhood to form particles of the ordered intermetallic palladium-gallium compound.

In more general terms, which of the constituent metals (other than palladium) of the Pd-hydrotalcite of the invention will form upon reduction the ordered intermetallic compound together with palladium in the Pd-hydrotalcite derived material of the invention depends on the (relative) standard reduction potentials $E^0$ of the constituent metals and the reduction conditions applied.

Those metal cations M2 and M3 (as defined in formula (I) above), which shall form together with palladium the particles of an ordered intermetallic compound should preferably have a significantly more positive standard reduction potential $E^0$ than those metal cations, which shall not be reduced and instead form part of the oxide matrix of the Pd-hydrotalcite derived material. As used herein, the standard reduction potentials $E^0$ are measured at 25°C and a pressure of 1 atm. In view of the above, $Mg^{2+}$ having a standard reduction potential $E^0$ as low as -2.372 V is a suitable M2 candidate when M3 shall be reduced to form together with Pd the ordered intermetallic palladium compound supported on the carrier. When it is desired that M2 forms together with palladium the ordered intermetallic compound, $Al^{3+}$ is a suitable cation M3 because it has a standard reduction potential as low as -1.662 V. Listings of standard reduction potentials are provided in common handbooks such as the CRC Handbook of Chemistry and Physics.

Stated more generally, the "ordered intermetallic compound of palladium and M2 and/or M3" in the Pd-hydrotalcite derived material as meant in Claims 8 and 9 can be understood as follows. It is an ordered intermetallic compound of palladium together with those M2 and/or M3 metal cation(s) (as defined in connection with formula (I) above) present in the Pd-hydrotalcite starting material, which can be reduced most easily amongst the M2 and M3 metal cations of the Pd-hydrotalcite starting material, i.e. which have a more positive standard reduction potential $E^0$ than the other metal cation(s) M2 and/or M3 of the Pd-hydrotalcite starting material.

Applying the above, it is readily understandable that a PdZnAl-hydrotalcite precursor will yield in the conversion method as recited in the appending Claim 8 a Pd-hydrotalcite derived material comprising particles of an ordered intermetallic palladium-zinc compound.

Concretely, intermetallic PdZn particles, in particular nanoparticles will form when the ratio of Pd and Zn in the PdZnAl-hydrotalcite precursor is about 1. Generally speaking, the ratio of the constituent metals of the Pd-hydrotalcite precursor, which, owing to their relative standard reduction potential $E^0$ (as explained above), will form upon reduction the ordered intermetallic compound comprised in the Pd-hydrotalcite derived material, will determine, which specific ordered intermetallic compound is formed.

In the materials obtainable upon reduction in the method of converting the Pd-hydrotalcite according to the present invention to Pd-hydrotalcite derived material, the particles of an ordered intermetallic compound of palladium and M2 and/or M3 may, depending on the reduction conditions, be supported on a carrier comprising oxides of those kinds of M2 and M3, which are, owing to their reduction potentials as explained above, not incorporated in the ordered intermetallic compound of the particles. A Pd-hydrotalcite derived material obtainable from a PdMgGa-hydrotalcite precursor will for example typically have a carrier or matrix comprising $Ga_2O_3$ and $MgO$ or $MgGa_2O_4$. This can be seen from XRD measurements.

The Pd-hydrotalcite derived materials according to the present invention are preferably free of elemental palladium. This could be confirmed by X-ray powder diffraction analysis (XRD). Since palladium has a low selectivity in selective hydrogenation reactions, the materials are therefore highly selective hydrogenation catalysts. Also, owing to the manufacturing method starting from Pd-hydrotalcite precursors, the particles of ordered intermetallic palladium compounds are finely distributed in the Pd-hydrotalcite derived material. Moreover, again due to the preparation method, the Pd-hydrotalcite derived material has a high porosity as indicated by a specific surface area (measured in accordance with the BET method, using nitrogen) as high as 70 to 160 $m^2/g$.

The Pd-hydrotalcite derived materials of the invention proved to be highly active and selective catalysts, for example in a process for the, preferably selective, hydrogenation of an alkyne(s) to give the corresponding alkene(s), also referred to as the semihydrogenation of the alkyne(s). A hydrogenation catalyst comprising a Pd-hydrotalcite derived material in accordance with the present invention, e.g. in a proportion of $\geq 20$ wt.-%, preferably $\geq 50$ wt.-%, more preferably $\geq 80$ wt.-%, still more preferably $\geq 90$ wt.-% and most preferably $\geq 95$ wt.-% was found to be highly selective to the desired alkene, in particular in the hydrogenation of acetylene (ethyne) to ethene, even when the ethyne is present in admixture with a large excess of ethene in the reaction mixture. According to a particularly preferred embodiment, the hydrogenation catalyst for use in the, preferably selective, hydrogenation of alkyne(s) to give the corresponding alkene (s) according to the present invention consists of a Pd-hydrotalcite derived material as meant herein.

Generally, a hydrogenation of an alkyne is referred to as selective if the triple bond is hydrogenated with preference only once, and the further reaction to the single bond is hardly observed, i.e. if the semihydrogenation of the triple bond is predominant. For the purpose of the present invention, the hydrogenation of an alkyne is referred to as selective if the molar ratio of the desired target compound, e.g. the corresponding alkene, to the undesired target compound, *e.g.* the corresponding alkane, is larger than 1 : 1, preferably more than 2 : 1, more preferably more than 5 : 1, and most preferably more than 10 : 1.

The alkyne to be converted in the selective hydrogenation process of the invention is for example an alkyne, dialkyne, trialkyne or polyalkyne. Preferably, it is an alkyne, i.e. a hydrocarbon compound containing only a single carbon-carbon triple bond. The alkyne to be subjected to the hydrogenation, preferably selective hydrogenation, in the present invention may have functional groups other than the carbon-carbon triple bond(s).

The alkyne is preferable ethyne (acetylene), and this is the most preferred embodiment of the present invention. Through the process for the selective hydrogenation of the invention, ethyne will predominantly be converted to ethene (ethylene) while the hydrogenation of ethene to afford ethane is negligible. This is even so when the selective hydrogenation of ethyne is carried out under reaction conditions where ethyne is present in admixture with an excess of ethene in relation to ethyne, which is a particularly preferred embodiment of the selective ethyne hydrogenation according to the present invention. Most preferably, ethene is present in the reaction mixture to be hydrogenated in a large excess in relation to ethyne. The ethyne to ethene weight ratio in the starting mixture of the selective ethyne hydrogenation of the invention is preferably 1 : 10 to 1 : $10^6$, more preferably 1 : 50 to 1 : $10^3$. In industrial processes, the ethene to ethyne weight ratio in the mixture obtained after the selective hydrogenation is typically as large as $> 10^6$.

The selective hydrogenation of phenyl acetylene to styrene in excess of styrene is another example of a selective hydrogenation of an alkyne. As will be appreciated, that reaction is the polystyrene counterpart of the selective acetylene hydrogenation in excess of ethylene in the feed used for the preparation of polyethylene.

EXAMPLES

Preparation of Pd-hydrotalcites

The nitrates of Pd(II), Mg(II) and Ga(III) were dissolved in molar ratios of Pd:Mg:Ga = x:70-x:30(x=0.0-5.0) in water to give a salt solution having a total metal salt concentration of 0.1 M. By adding a mixed aqueous $NaOH/Na_2CO_3$ solution with a total concentration of 0.345 M, or a pure 0.345 M aqueous $Na_2CO_3$ solution, the Pd-hydrotalcite was precipitated, as follows.

The above 0.1 M metal salt solution (pH 0-1) was fed at a constant rate (16 g/min) into a 2 L stirred reactor containing 300 mL $H_2O$ or highly diluted aqueous $Na_2CO_3$ solution (pH 8.5). Then, the basic precipitating agent (0.345 M aqueous $NaOH/Na_2CO_3$ solution or 0.345 M aqueous $Na_2CO_3$ solution) was charged by an automatic feedback loop (laboratory reactor LabMax, Mettler Toledo) such that the pH in the reactor remained constant near 8.5 after an induction phase of < 5 min. After 40 min, the precipitation was stopped and the brown precipitate was aged under stirring in the mother liquor for 60 min. The temperature inside the reactor was 55°C during precipitation and aging. After aging, the precipitate was separated from the mother liquor by filtration and subsequently washed by repeated suspending in 400 mL deionised water. The washing was continued until the electric conductivity of the washing water became below 0.5 mS/cm. Subsequently, the product was dried in a muffle-type furnace at 80 °C for 12 hours.

Following the above protocol, a set of Pd-hydrotalcite samples with different nominal palladium loadings was prepared.

Characterization of Pd-hydrotalcites

The products were subjected to X-ray powder diffraction (XRD) analyses to verify that they have a hydrotalcite-like structure. XRD patterns were obtained, which closely resembled the theoretical pattern of MgGa-hydrotalcite (see Fig. 3). Moreover, the homogeneous distribution of palladium in the hydrotalcite structures was confirmed by means of scanning electron microscopy (SEM). Also, the thermal properties of the products were examined by thermogravimetric-mass spectroscopic analysis (TG-MS). Thermal properties, which are typical for hydrotalcite-like materials were found. Thus, the products were confirmed to be PdMgGa-hydrotalcites.

Conversion to Pd-hydrotalcite derived material

After drying as detailed above, the Pd-hydrotalcite samples were reduced in a flow of 5% hydrogen in argon at a specific temperature within a range of 600 to 800 °C (heating rate: 2 °C/min, 30 min holding time) to give Pd-hydrotalcite derived materials. By way of transmission electron microscopy (TEM) and high resolution TEM (HRTEM) and/or XRD it was confirmed that the obtained materials comprise nanoparticles of ordered palladium gallium intermetallic compounds.

Catalytic testing

The Pd-hydrotalcite derived materials were tested as catalysts in the selective hydrogenation of acetylene to ethylene in an excess of ethylene under the following conditions.

Catalytic tests were carried out in a plug flow reactor consisting of a quartz tube with a length of 300 mm, an inside diameter of 7 mm and equipped with a sintered glass frit to support the catalyst bed. The reaction temperature was generally 200 °C. For temperature control, a thermocouple was provided in the oven around the reactor. A second thermocouple was placed inside the reactor to measure the temperature of the catalyst bed. The reactant gases were mixed with Bronkhorst mass flow controllers (total flow 30 ml/min). A Varian CP 4900 Micro gas chromatograph (GC) was used for effluent gas analysis. The Varian MicroGC contains three modules, each with an individual column and a thermal conductivity detector. Hydrogen and helium of the feed gas, and possible oxygen and nitrogen impurities because of leaks in the set-up were separated on a molsieve column. Acetylene, ethylene, and ethane were separated on an alumina column. The total concentration of $C_4$ hydrocarbons (1-butyne, 1-butene, 1,3-butadiene, n-butane, trans and cis-2-butene) was determined using a siloxane (dimethylpolysiloxane) column. Higher hydrocarbons were also separated on the siloxan column but not further quantified because of the presence of many different $C_6$ and $C_8$ hydrocarbons and their low total concentration (less than 0.1% of absolute product stream concentration). Argon (6.0) and helium (6.0) were used as carrier gases for the molsieve column and for the other columns, respectively. A measurement cycle including stabilization, sampling, injection, and separation took between 4 and 5 minutes.

Acetylene hydrogenation experiments were carried out under the condition of 0.5% acetylene, 5% hydrogen, and 50% ethylene in helium. Gases were obtained from Westfalen Gas or Praxair (Germany). The conversion was calculated using the following equation:

$$Conv = \frac{(C_{bypass} - C_x)}{C_{bypass}}$$

where $C_x$ is the acetylene concentration in the product stream and $C_{bypass}$ is the acetylene concentration in the feed before the reaction. The selectivity was calculated from the following equation, with $C_{bypass}$ being the acetylene concentration before the reactor and $C_x$ the acetylene concentration after the reactor:

$$Sel = \frac{(C_{bypass} - C_x)}{C_{bypass} - C_x + C_{ethane} + 2xC_{C4Hx}}$$

Calculation of the selectivity assumes that acetylene is only hydrogenated to ethylene, which may be further hydrogenated to ethane. The amount of $C_6$ and higher hydrocarbons, and of carbon deposits formed was found to be negligible. In addition to hydrogenation of acetylene to ethane, ethylene from feed may be hydrogenated to ethane, which is included in the selectivity equation.

Activity of the samples was calculated using the following equation:

$$Act = \frac{Conv\ C_{feed}\ C_{exp}}{m_{cat}} \quad ,$$

wherein Conv is the calculated acetylene conversion, $C_{feed}$ is the concentration of acetylene in feed, i.e. 0.5 %, $m_{cat}$ the amount of used catalyst in g , and constant $C_{exp}$ is 1.904 g/h and contains experimental parameters like total gas flow (30 ml/min), temperature (300 K) and pressure (1013 mbar) and is based on the perfect gas model.

The samples were diluted with 150 mg boron nitride (hexagonal, 99.5%, 325 mesh, Aldrich) prior to conducting the catalytic tests.

Results of the catalytic testing of the Pd-hydrotalcite derived materials in the selective hydrogenation of acetylene under the above conditions are summarized in Table 1, below.

Table 1

| Ex. No. | Sample No. | Content of Pd in catalyst [mol%] * | Amount of cat [μg] | Reduction Temp. [°C] | Acetylene Conversion [%] ** | Selectivity [%]** | Activity [$g_{C2H2}/g_{Pdh}$] |
|---|---|---|---|---|---|---|---|
| 1 | #7945 | 3.1 | 25 | 600 | 60 | 65 | 6860.9 |
| 2 | #7946 | 3.1 | 55 | 700 | 78 | 68 | 3893.1 |
| 3 | #8191 | 3.1 | 52 | 800 | 78 | 71 | 4244.2 |
| 4 | #8330 | 4.9 | 20 | 600 | 71 | 68 | 7391.3 |
| * Determined by elemental analysis as [Pd] / ([Pd] + [Mg] + [Ga] ) <br> **After 18 h on stream | | | | | | | |

The XRD pattern of the Pd-hydrotalcite derived material of Example 1 is shown in Fig. 5 (confirming the presence of $Pd_2Ga$), and the catalytic properties (conversion and selectivity to ethylene) of the Pd-hydrotalcite derived material of Example 2 are additionally illustrated in Fig. 4.

Hence, it could be shown that the Pd-hydrotalcite derived materials of the invention, e.g. those comprising ordered intermetallic palladium gallium particles, in particular nanoparticles are highly active and selective catalysts, e.g. in the selective hydrogenation of acetylene to ethylene even when an excess of ethylene is present.

**Claims**

1. A hydrotalcite-like compound, wherein $Pd^{2+}$ occupies at least part of the octahedral sites in the brucite-like layers.

2. The hydrotalcite-like compound according to Claim 1, wherein 0.01 to 5 % of the octahedral sites in the brucite-like layers are occupied by $Pd^{2+}$.

3. The hydrotalcite-like compound according to Claim 1, which is a Pd-hydrotalcite represented by the following formula:

$$[(Pd^{2+}, M2)_{1-x} M3_x (OH)_2]^{x+} (A^{n-}_{x/n}) \cdot m \, H_2O$$

wherein

   M2 is at least one divalent metal cation selected from the group consisting of $Mg^{2+}$, $Ni^{2+}$, $CO^{2+}$, $Zn^{2+}$, $Fe^{2+}$, $Cu^{2+}$ and $Mn^{2+}$;
   M3 is at least one trivalent metal cation selected from $Al^{3+}$, $Ga^{3+}$, $Ni^{3+}$, $Co^{3+}$, $Fe^{3+}$, $Mn^{3+}$ und $Cr^{3+}$;
   A is an n-valent anion, preferably carbonate;
   x is 0.1 - 0.5, preferably $0.2 \leq x \leq 0.33$; and
   m is 0.1-1.0.

4. The hydrotalcite-like compound according to Claim 3, wherein
   M2 is $Mg^{2+}$ and M3 is $Ga^{3+}$; or
   M2 is $Zn^{2+}$ and M3 is $Al^{3+}$.

5. The hydrotalcite-like compound according to Claim 3, wherein M3 is $Ga^{3+}$.

6. A method of preparing a hydrotalcite-like compound as defined in any one of Claims 3 to 5, comprising the dissolution of water-soluble salts of Pd(II), M2 and M3 in an aqueous solvent, and the addition of the anion(s) A to precipitate the hydrotalcite-like compound.

7. The method according to Claim 6, wherein the precipitated hydrotalcite-like compound is, after optional ageing, separated from the solution, dried and optionally subjected to calcination.

8. A method of converting a hydrotalcite-like compound as defined in any one of Claims 3 to 5 into a material comprising particles of an ordered intermetallic compound of palladium and M2 and/or M3, which method comprises the reduction of the hydrotalcite-like compound at temperatures in the range of 100-1000°C, preferably 500-800 °C.

9. A material obtainable by the method of Claim 8.

10. The material according to Claim 9, wherein the ordered intermetallic compound is PdGa, $Pd_2Ga$ or PdZn.

11. The material according to Claim 9 or 10, wherein the particles of the ordered intermetallic compound are nanoparticles.

12. A use of the material according to any one of Claims 9 to 11 as a catalyst.

13. A process for the, preferably selective, hydrogenation of alkyne(s) to give the corresponding alkene(s), which process comprises reacting a reaction mixture comprising the alkyne(s) with hydrogen in the presence of a hydrogenation catalyst, wherein the hydrogenation catalyst comprises a material as defined in any one of Claims 9 to 11.

14. The process according to Claim 13, wherein the alkyne is ethyne which is converted to ethene through the selective hydrogenation.

**15.** The process according to Claim 14, wherein the ethyne is present in admixture with an excess of ethene in the reaction mixture.

## Fig. 1

Fig. 2a

7

Fig. 2b

7

1

Fig. 3

Fig. 4

Fig. 5

Fig. 6

Application Number

EP 10 00 2419

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FRANCOVA ET AL: "Hydrogenation of 2-butyne-1,4-diol on supported Pd catalysts obtained from LDH precursors" MICROPOROUS AND MESOPOROUS MATERIALS, ELSEVIER SCIENCE PUBLISHING, NEW YORK, US LNKD- DOI:10.1016/J.MICROMESO.2006.07.038, vol. 99, no. 1-2, 18 January 2007 (2007-01-18), pages 118-125, XP005823588 ISSN: 1387-1811 | 1-3,6-9, 11,12 | INV. C01F7/00 B01J23/44 C01G55/00 B01J23/60 B01J23/62 B01J37/16 C07C5/05 C07C5/09 C22C5/04 |
| Y | * the whole document * | 4,5 | |
| X | TICHIT ET AL: "Design of nanostructured multifunctional Pd-based catalysts from layered double hydroxides precursors" APPLIED CATALYSIS A: GENERAL, ELSEVIER SCIENCE, AMSTERDAM, NL LNKD- DOI:10.1016/J.APCATA.2006.10.052, vol. 318, 25 January 2007 (2007-01-25), pages 170-177, XP005834988 ISSN: 0926-860X | 1-3,6-9, 11,12 | |
| | | | TECHNICAL FIELDS SEARCHED (IPC) |
| Y | * the whole document * | 4,5 | C01F B01J C01G C07C C22C |
| Y | WO 2006/066341 A1 (UNIV QUEENSLAND [AU]; LU GAOQING [AU]; XU ZHIPING [AU]) 29 June 2006 (2006-06-29) * page 3, lines 21-32 * | 4,5 | |
| A,D | WO 2009/062848 A2 (MAX PLANCK GESELLSCHAFT [DE]; ARMBRUESTER MARC [DE]; SCHMIDT MARCUS [D) 22 May 2009 (2009-05-22) * claims * | 1-15 | |
| A,D | WO 2009/037301 A1 (MAX PLANCK GESELLSCHAFT [DE]; ARMBRUESTER MARC [GB]; SCHMIDT MARCUS [D) 26 March 2009 (2009-03-26) * claims * | 1-15 | |

-/--

The present search report has been drawn up for all claims

| Place of search | Date of completion of the search | Examiner |
|---|---|---|
| Munich | 6 August 2010 | Stratford, Katja |

EPO FORM 1503 03.82 (P04C01)

Europäisches
Patentamt

European
Patent Office

Office européen
des brevets

# EUROPEAN SEARCH REPORT

Application Number

EP 10 00 2419

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | MONZON A ET AL: "Use of hydrotalcites as catalytic precursors of multimetallic mixed oxides. Application in the hydrogenation of acetylene" APPLIED CATALYSIS A: GENERAL, ELSEVIER SCIENCE, AMSTERDAM, NL LNKD-DOI:10.1016/S0926-860X(99)00101-5, vol. 185, no. 1, 6 September 1999 (1999-09-06), pages 53-63, XP004271907 ISSN: 0926-860X * the whole document * ----- | 1-15 | |

TECHNICAL FIELDS SEARCHED (IPC)

The present search report has been drawn up for all claims

| Place of search | Date of completion of the search | Examiner |
|---|---|---|
| Munich | 6 August 2010 | Stratford, Katja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 00 2419

This annex lists the patent family members relating to the patent documents cited in the above-mentioned European search report.
The members are as contained in the European Patent Office EDP file on
The European Patent Office is in no way liable for these particulars which are merely given for the purpose of information.

06-08-2010

| Patent document cited in search report | | Publication date | Patent family member(s) | | Publication date |
|---|---|---|---|---|---|
| WO 2006066341 | A1 | 29-06-2006 | CA | 2594132 A1 | 29-06-2006 |
| | | | CN | 101128626 A | 20-02-2008 |
| | | | EP | 1838906 A1 | 03-10-2007 |
| | | | JP | 2008525292 T | 17-07-2008 |
| | | | US | 2009108233 A1 | 30-04-2009 |
| WO 2009062848 | A2 | 22-05-2009 | EP | 2060323 A1 | 20-05-2009 |
| WO 2009037301 | A1 | 26-03-2009 | EP | 2039669 A1 | 25-03-2009 |
| | | | EP | 2197819 A1 | 23-06-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007104569 A
- EP 1834939 A
- WO 2009037301 A
- EP 2039669 A
- EP 2060323 A
- WO 2009062848 A
- DE 2024282 A

### Non-patent literature cited in the description

- **J. OSSWALD.** *J. of Catal.,* 2008, vol. 258, 210
- **J. OSSWALD et al.** *J. of Catal.,* 2008, vol. 258, 219
- **T. KOMATSU et al.** *Appl. Catal. A,* 2003, vol. 251, 315
- **F. CAVANI et al.** *Catal. Today,* 1991, vol. 11, 173
- **E. LOPÉZ-SALINAS et al.** *J. Phys. Chem. B.,* 1997, vol. 101, 5112
- *J. Porous Mater.,* 1996, vol. 3, 169
- **A. MONZÓN.** *Appl. Catalysis A,* 1999, vol. 185, 53
- **F. CAVANI.** *Catalysis Today,* 1991, vol. 11, 173-301